# EUROPEAN PATENT APPLICATION

(11) **EP 2 689 712 A1**
(43) Date of publication of application: **29.01.2014**
(21) Application number: 13176677.6
(22) Date of filing: 16.07.2013
(51) Int. Cl.: A61B 1/00, A61B 1/06

(54) **Endoscope system, processor device of endoscope system, and method for controlling display of endoscope image**

(30) Priority: 24.07.2012 JP 2012164230
(71) Applicant: Fujifilm Corporation, Minato-ku Tokyo (JP)
(72) Inventor: Kaku, Toshihiko, Ashigarakami-gun Kanagawa (JP)
(74) Representative: Klunker . Schmitt-Nilson . Hirsch

(57) **Abstract**

Illumination light for highlighting vessels and measurement light for measuring oxygen saturation level is applied alternately to a subject. A color image sensor (60) images the subject illuminated with the illumination light and acquires a first blue signal, a first green signal, and a first red signal. The color image sensor images the subject illuminated with the measurement light and acquires a second blue signal, a second green signal, and a second red signal. An oxygen saturation level of hemoglobin in blood is calculated using the second blue signal, the first green signal, and the first red signal. The first blue signal changed in accordance with the oxygen saturation level, the first green signal, and the first red signal are assigned to respective B, G, and R channels of a display (14). The display displays a "high-contrast vessel image with oxygen saturation level" that varies with the oxygen saturation level.

## Description

### BACKGROUND OF THE INVENTION

### 1. Field of the invention

The present invention relates to an endoscope system, a processor device of an endoscope system, and a method for controlling a display of an endoscope image, capable of displaying an enhanced blood vessel pattern and an oxygen saturation level of hemoglobin in blood.

### 2. Description Related to the Prior Art

Cancer diagnoses using endoscope systems have been common in the medical field. The endoscope system comprises a light source device, a processor device, and an endoscope device. To perform cancer diagnosis, first, an insert section of the endoscope device is inserted into a body cavity. From a distal portion of the insert section, illumination light of predetermined wavelengths is applied to a region of interest in the body cavity. An image sensor disposed in the distal portion images the region of interest illuminated and thus a color image carrying various types of biological information is obtained. For example, in Japanese Patent No. 3559755, narrowband light of predetermined wavelengths is used as the illumination light. Thereby surface blood vessels and surface microstructure, which are difficult to observe with broadband illumination light such as white light, are highlighted or enhanced in a display. The display of a blood vessel pattern with the clear surface blood vessels and the like allows determination of cancer and estimation of its invasion depth.

In Japanese Patent No. 2648494, an oxygen saturation level of hemoglobin in blood is imaged with the use of illumination light including a wavelength range in which an absorption coefficient of oxyhemoglobin differs from that of deoxyhemoglobin. In an image showing the oxygen saturation levels, lesion sites, such as cancer, in hypoxic (low oxygen) conditions are displayed in colors different from those of normal sites in hyperoxic (high oxygen) conditions. Thus, distribution of the cancer is intuitively recognized from a high-contrast vessel image displayed with the oxygen saturation levels.

It may be difficult to diagnose cancer only from a blood vessel pattern with surface capillary vessels and the like highlighted as described in the Japanese patent No. 3559755, depending on its pattern. On the other hand, it is rather easy to diagnose the cancer from the image showing oxygen saturation levels as described in the Japanese Patent No. 2648494. However, the information of the oxygen saturation levels may be insufficient to further diagnose the invasion depth of the cancer, or the like. To improve diagnosing performance for cancer, it is necessary to highlight or enhance the blood vessel pattern specific to cancer, for example, surface capillary vessels, in a display and to display oxygen conditions of the blood vessels together with the blood vessel pattern.

### SUMMARY OF THE INVENTION

An object of the present invention is to provide an endoscope system, a processor device of an endoscope system, and a method for controlling a display of an endoscope image, capable of highlighting or enhancing a blood vessel pattern such as surface capillary vessels in a display and displaying an oxygen saturation level of hemoglobin in blood together with the blood vessel pattern.

To achieve the above and other objects, an endoscope system comprises a lighting means, an image signal acquisition means, a display control means, and a display means. The lighting means applies each of first illumination light and second illumination light on a frame-by-frame basis to a subject including a blood vessel. The first illumination light includes at least one type of narrowband light. The second illumination light includes a wavelength range in which an absorption coefficient varies with a change in oxygen saturation level of hemoglobin in blood. The image signal acquisition means images the subject, illuminated with the first illumination light, on the frame-by-frame basis to acquire a first color image signal and images the subject, illuminated with the second illumination light, on the frame-by-frame basis to acquire a second color image signal. The display control means produces a display image in which at least a first color signal, out of the first color image signal, is changed in accordance with the oxygen saturation level obtained using at least the second color image signal. The display means displays the display image.

It is preferable that the at least one type of narrowband light is at least one of blue narrowband light and green narrowband light.

It is preferable that the first color image signal includes a blue signal, a red signal, and a green signal and one of the blue signal, the red signal, and the green signal is the first color signal. It is preferable that the display means has B, G, and R channels.

It is preferable that the display control means changes the blue signal which is to be assigned to the B channel in accordance with the oxygen saturation level. The blue signal which is to be assigned to the B channel is the first color signal. It is preferable that the display control means does not change the blue signal which is to be assigned to the G channel and the green signal which is to be assigned to the R channel.

It is preferable that the display control means changes the blue signal which is to be assigned to the G channel in accordance with the oxygen saturation level. The blue signal which is to be assigned to the G channel is the first color signal. It is preferable that the display control means does not change the blue signal which is to be assigned to the B channel and the green signal which is to be assigned to the R channel.

It is preferable that the display control means changes the red signal which is to be assigned to the R channel in accordance with the oxygen saturation level. The red signal which is to be assigned to the R channel is the first color signal. It is preferable that the display control means does not change the blue signal which is to be assigned to the B channel and the green signal which is to be assigned to the G channel.

It is preferable that the at least first color signal is two of the blue signal, the red signal, and the green signal.

It is preferable that the endoscope system further comprises an oxygen saturation calculation means for calculating the oxygen saturation level from the second color image signal or the first and the second color image signals.

It is preferable that the endoscope system further comprises a vessel depth determination means for determining the depth of a blood vessel in the subject based on the first or second color image signal. The display control means changes the first color signal in accordance with the depth of the blood vessel and the oxygen saturation level to control the display image.

It is preferable that the display control means changes the first color signal in accordance with the oxygen saturation level when the oxygen saturation level is less than a predetermined value or a standardization signal is out of a predetermined range. The standardization signal is obtained by standardizing the second color image signal with the first color image signal.

It is preferable that the endoscope system comprises a mode selection means for selecting one of two modes. The two modes include at least one of a narrowband image plus oxygen saturation mode, a narrowband mode, and a normal image plus oxygen saturation mode. The display image is displayed in the narrowband image plus oxygen saturation mode. A narrowband image produced based on the first color image signal is displayed in the narrowband mode. An image in which a color characteristic value of a normal image is changed in accordance with the oxygen saturation level is displayed in the normal image plus oxygen saturation mode. The normal image is produced by imaging the subject illuminated with broadband third illumination light.

It is preferable that the image signal acquisition means has a color image sensor. It is preferable that the first illumination light includes blue narrowband light of 440 to 460 nm, fluorescence obtained by converting the blue narrowband light of 440 to 460 nm with a wavelength converter, and blue narrowband light with a center wavelength of 400 to 410 nm. It is preferable that the second illumination light includes blue narrowband light of 460 to 480 nm and fluorescence obtained by converting the blue narrowband light of 460 to 480 nm with a wavelength converter.

It is preferable that the image signal acquisition means has a monochrome image sensor. It is preferable that the first illumination light includes blue light of 400 to 420 nm and green light of 530 to 550 nm applied alternately. It is preferable that the second illumination light is blue light of 450 to 500 nm.

A processor device, used in an endoscope system, comprises a receiving means and a display control means. The receiving means receives the first and second color image signals from the endoscope device. The display control means produces a display image in which at least a first color signal, out of the first color image signal, is changed in accordance with an oxygen saturation level obtained using the at least second color image signal, and displays the display image on a display means. The endoscope system includes a lighting device and an endoscope device. The lighting device applies each of first illumination light and second illumination light on a frame-by-frame basis to a subject including a blood vessel. The first illumination light includes narrowband light. The second illumination light includes a wavelength range in which an absorption coefficient varies with a change in oxygen saturation level of hemoglobin in blood. The endoscope device images the subject, illuminated with the first illumination light, on the frame-by-frame basis to acquire a first color image signal. The endoscope device images the subject, illuminated with the second illumination light, on the frame-by-frame basis to acquire a second color image signal.

A method for controlling a display of an endoscope image, comprises a signal acquiring step, a producing step, and a displaying step. In the signal acquiring step, each of first illumination light and second illumination light is applied on a frame-by-frame basis to a subject including a blood vessel. The first illumination light includes narrowband light. The second illumination light includes a wavelength range in which an absorption coefficient varies with a change in oxygen saturation level of hemoglobin in blood. The subject illuminated with the first illumination light is imaged with an endoscope device on the frame-by-frame basis and thereby a first color image signal is acquired. The subject illuminated with the second illumination light is imaged with the endoscope device on the frame-by-frame basis and thereby a second color image signal is acquired. In the producing step, a display image in which at least a first color signal, out of the first color image signal, is changed in accordance with an oxygen saturation level is produced. The oxygen saturation level is obtained using at least the second color image signal. In the displaying step, the display image is displayed on a display means.

According to the present invention, the blood vessel pattern such as the surface capillary vessels is highlighted and displayed. The oxygen saturation level of hemoglobin in blood is displayed together with the blood vessel pattern.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Fig. 1 is an external view of an endoscope system;
Fig. 2 is a block diagram illustrating an internal configuration of an endoscope system according to a first embodiment;
Fig. 3A is a graph illustrating an emission spectrum of normal light in a normal mode;
Fig. 3B is a graph illustrating an emission spectrum of illumination light for highlighting blood vessels in a narrowband mode;
Fig. 3C illustrates emission spectra of measurement light for measuring oxygen saturation level and illumination light for highlighting blood vessels in a narrowband image plus oxygen saturation mode;
Fig. 4A illustrates B, G, and R pixels in a color image sensor;
Fig. 4B is a graph illustrating spectral transmittance of B, G, and R pixels;
Fig. 5A is an explanatory view of imaging control of the image sensor in the normal mode of the first embodiment;
Fig. 5B is an explanatory view of imaging control of the image sensor in the narrowband mode of the first embodiment;
Fig. 5C is an explanatory view of imaging control of the image sensor in the narrowband image plus oxygen saturation mode of the first embodiment;
Fig. 6 is a block diagram illustrating a function of an image processor;
Fig. 7 is an explanatory view of color allocation in the narrowband mode;
Fig. 8 is a graph illustrating a correlation between the oxygen saturation level and the signal ratios B1/G2 and R2/G2;
Fig. 9 is a graph illustrating absorption coefficients of hemoglobin;
Fig. 10 is an explanatory view illustrating how to obtain an oxygen saturation level from the signal ratios B1^{*}/G2^{*} and R2^{*}/G2^{*} using the graph of Fig. 8;
Fig. 11 is a graph illustrating relationships between the signal ratio B2/G2 and surface blood vessels, mucosa, and subsurface blood vessels;
Fig. 12 is an explanatory view illustrating color allocation in the pixels corresponding to the surface blood vessels;
Fig. 13 is a graph illustrating a relationship between a gain increase and the oxygen saturation level;
Fig. 14 is an explanatory view of a color of the displayed surface blood vessels in a hyperoxic (high oxygen) condition;
Fig. 15 is an explanatory view of a color of the displayed surface blood vessels in a hypoxic (low oxygen) condition;
Fig. 16 is an explanatory view of color allocation in pixels corresponding to the subsurface blood vessels;
Fig. 17 is a graph illustrating a relationship between gain reduction and the oxygen saturation level;
Fig. 18 is an explanatory view of a color of the displayed subsurface blood vessels in the hyperoxic condition;
Fig. 19 is an explanatory view of a color of the displayed subsurface blood vessels in the hypoxic condition;
Fig. 20 is a flowchart illustrating steps in the narrowband mode and steps in the narrowband image plus oxygen saturation mode;
Fig. 21A is an explanatory view illustrating differences in color of the displayed blood vessels with high oxygen saturation levels between the modes;
Fig. 21B is an explanatory view illustrating differences in color of the displayed blood vessels with low oxygen saturation levels between the modes;
Fig. 22 is a block diagram illustrating an internal configuration of an endoscope system of a second embodiment;
Fig. 23 is a front view of a rotation filter;
Fig. 24 is a graph illustrating transmittance of each filter portion of the rotation filter;
Fig. 25A is an explanatory view of imaging control of the image sensor in a normal mode of the second embodiment;
Fig. 25B is an explanatory view of imaging control of the image sensor in the narrowband mode of the second embodiment;
Fig. 25C is an explanatory view of imaging control of the image sensor in the narrowband image plus oxygen saturation mode of the second embodiment;
Fig. 26 is an explanatory view of color allocation in a third embodiment;
Fig. 27 is an explanatory view of colors of the surface and subsurface blood vessels in the hyperoxic condition;
Fig. 28 is an explanatory view of colors of the surface and subsurface blood vessels in the hypoxic condition; and
Fig. 29 is a graph illustrating the relationship between gain increase and the signal ratio B1/G2.

### DESCRIPTION OF THE PREFERRED EMBODIMENTS

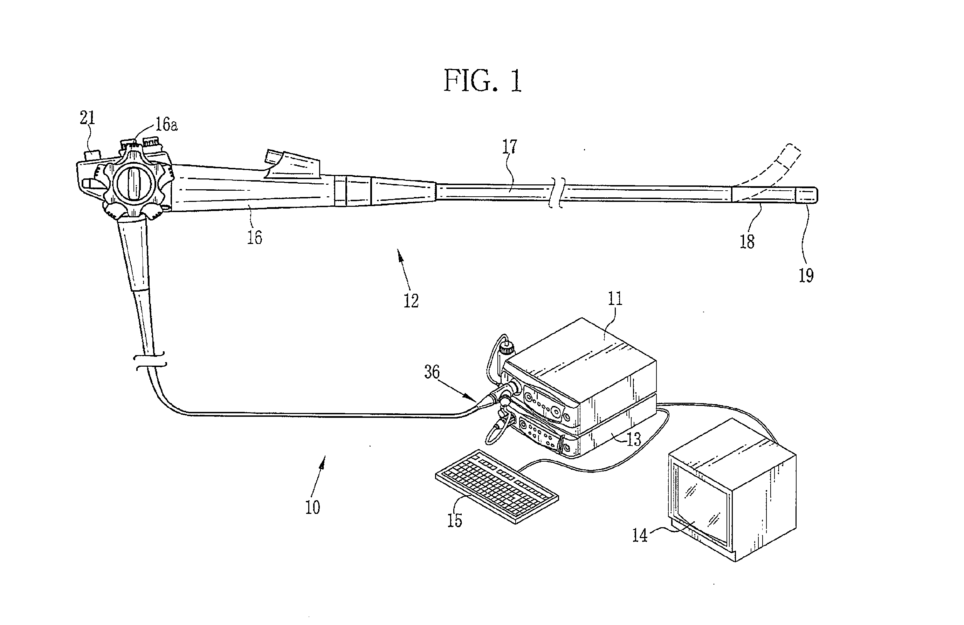

As shown in Fig. 1, an endoscope system 10 of a first embodiment is provided with a light source device 11, an endoscope device 12, a processor device 13, a display device 14, and an input device 15. The light source device 11 generates light to illuminate inside of a subject. The endoscope device 12 applies the light from the light source device 11 to a region of interest of the subject and captures a reflected image. Thereby, the endoscope device 12 outputs a color image signal. The processor device 13 acquires the color image signal from the endoscope device 12 and performs image processing on the color image signal to produce an endoscope image. The display device 14 displays the endoscope image and the like. The input device 15 is composed of a keyboard and the like.

The endoscope device 12 is provided with a flexible tube portion 17, a bending portion 18, and a distal portion 19 in this order from a handling section 16 side. The bending portion 18 is bent by rotating an angle knob 16a disposed on the handling section 16. The bending portion 18 can be bent at any desired angle and in any desired direction to direct the distal portion 19 toward a region of interest.

The endoscope system 10 has a normal mode, a narrowband mode, and a narrowband image plus oxygen saturation mode. In the normal mode, a normal image is displayed on the display device 14. The normal image is a subject image of visible light in a wavelength range from blue to red. In the narrowband mode, a high-contrast vessel image is displayed on the display device 14. The high-contrast vessel image includes a highlighted blood vessel image in which surface blood vessels and subsurface blood vessels in the subject are highlighted or enhanced. In the narrowband image plus oxygen saturation mode, a high-contrast vessel image with oxygen saturation level is displayed on the display device 14. The high-contrast vessel image with oxygen saturation level is a highlighted blood vessel image with its color (s) changed in accordance with oxygen saturation level(s) of hemoglobin in blood. These three modes are switched using a changeover switch 21, the input device 15, or the like.

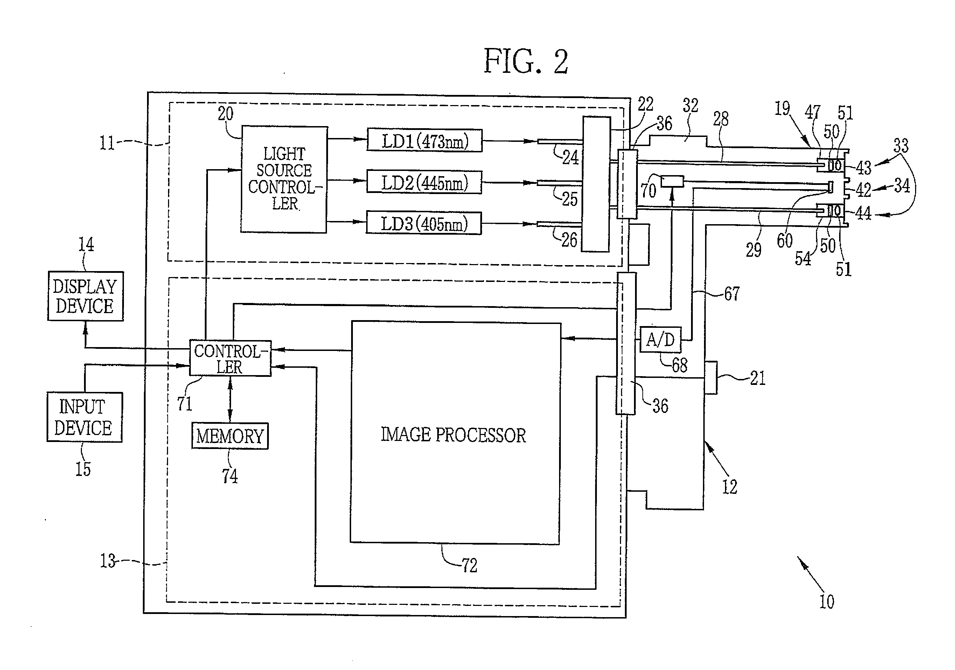

As shown in Fig. 2, the light source device 11 comprises three types of lasers LD1, LD2, and LD3, and a light source controller 20. The laser LD1 emits first laser beams with the center wavelength of 473 nm. A phosphor 50 (wavelength converter) converts the first laser beams into fluorescence in a wavelength range of green to red. The phosphor 50 is disposed in the distal portion 19 of the endoscope device 12. The laser LD2 emits second laser beams with the center wavelength of 445 nm. The phosphor 50 converts the second laser beams into fluorescence. The third laser LD3 emits third laser beams with the center wavelength of 405 nm. The phosphor 50, disposed in the distal portion 19, absorbs only a part of the third laser beams and converts the absorbed third laser beams into fluorescence. Most of the third laser beams pass through the phosphor 50 without being absorbed. The first, second, and third laser beams are incident on optical fibers 24, 25, and 26 through condenser lenses (not shown), respectively.

Note that the first laser beams are preferably in a wavelength range of 460 to 480 nm. The second laser beams are preferably in a wavelength range of 440 to 460 nm. The third laser beams is preferably in a wavelength range of 400 to 410 nm. The lasers LD1, LD2, and LD3 may be broad area type InGaN laser diodes, InGaNAs laser diodes, or GaNAs laser diodes, for example.

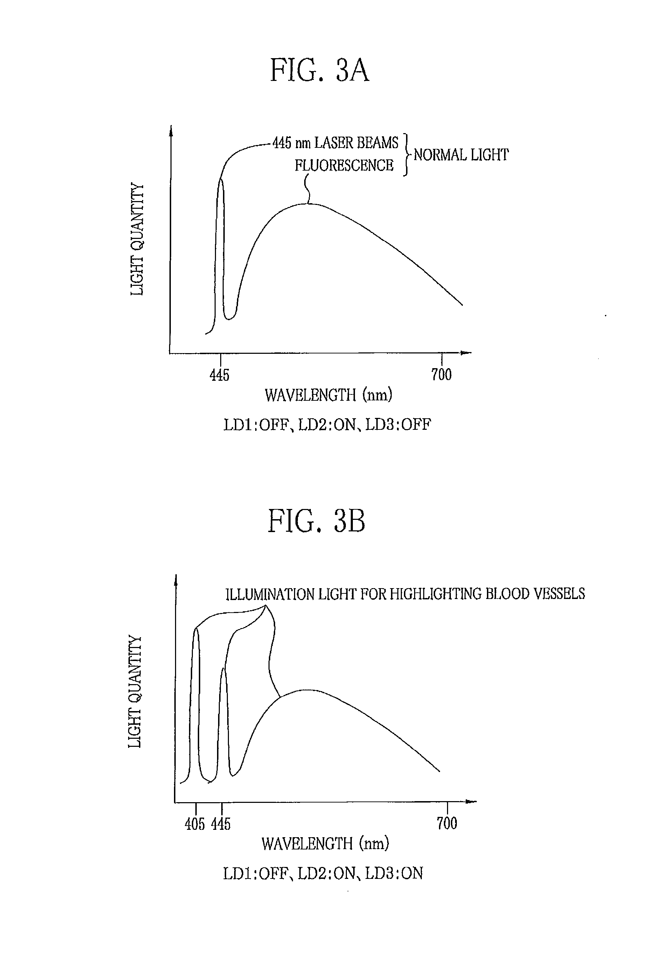

The light source controller 20 controls lasers LD1 to LD3. As shown in Fig. 3A, in the normal mode, the laser LD2 is turned on while the lasers LD1 and LD3 are turned off. Hence, the normal light, composed of the second laser beams from the laser LD2 and the fluorescence from the phosphor 50 excited by the second laser beams, is applied to the subject. As shown in Fig. 3B, in the narrowband mode, the lasers LD2 and LD3 are turned on while the laser LD1 is turned off. Thereby, illumination light for highlighting blood vessels (hereinafter simply referred to as the highlighting light) composed of the normal light and the third laser beams from the third laser LD3 is applied to the subject.

In Fig. 3B, to highlight the surface blood vessels having high absorption characteristics in a blue region, note that a light quantity of the third laser beams (emission peak: 405 nm) is greater than that of the second laser beams (emission peak: 445 nm) in the highlighting light. To highlight the subsurface blood vessels having high absorption characteristics in a green region, on the contrary, it is preferable to make the light quantity of the second laser beams greater than that of the third laser beams to increase the light quantity of fluorescence. Note that a ratio between the light quantities of the second and third laser beams is adjusted using the light source controller 20.

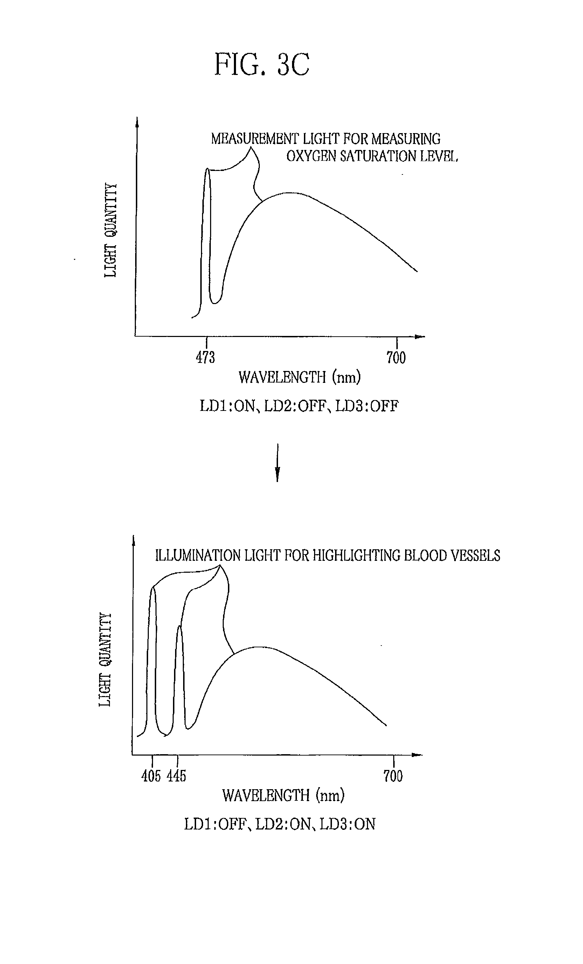

As shown in Fig. 3C, in the narrowband image plus oxygen saturation mode, emission is controlled to repeat a first emission pattern and a second emission pattern alternately. In the first emission pattern, the laser LD1 is turned on while the second and third lasers LD2 and LD3 are turned off. Thereby, measurement light for measuring oxygen saturation level (hereinafter simply referred to as the measurement light) is applied to the subject. The measurement light includes the first laser beams from the laser LD1 and the fluorescence from the phosphor 50 excited by the first laser beams. In the second emission pattern, the lasers LD2 and LD3 are turned on while the laser LD1 is turned off. Thereby, the highlighting light is applied to the subject. The measurement light and the highlighting light is applied alternately to the subject.

As shown in Fig. 2, a splitter 22 splits each of the first laser beams from the optical fiber 24, the second laser beams from the optical fiber 25, and the third laser beams from the optical fiber 26 into two paths. The two paths of laser beams are incident on respective light guides 28 and 29. Each of the light guides 28 and 29 is composed of a fiber bundle of optical fibers.

The endoscope device 12 is composed of an electronic endoscope. The endoscope device 12 comprises a lighting section 33, an imaging section 34, and a connector 36. The lighting section 33 applies the two paths of light, transmitted through the light guides 28 and 29, to the region of interest. The imaging section 34 images the region of interest. The connector 36 connects the endoscope device 12, the light source device 11, and the processor device 13 in a detachable manner.

The lighting section 33 comprises two lighting windows 43 and 44. The imaging section 34 is located between the lighting windows 43 and 44. Each of the lighting windows 43 and 44 applies the light passed through the phosphor 50 to the region of interest. The imaging section 34 comprises a capture window 42 located at the approximate center of the distal portion 19. The capture window 42 receives reflection light reflected from the region of interest.

Projection units 47 and 54 are accommodated behind the lighting windows 43 and 44, respectively. The projection unit 47 applies the first, second, or third laser beams, being excitation light, from the light guide 28 to the phosphor 50. The projection unit 54 applies the first, second, or third laser beams, being the excitation light, from the light guide 29 to the phosphor 50. Thereby, the phosphor 50 emits fluorescence. The first, second, or third laser beams and the fluorescence are applied to the region of interest through a lens 51.

The phosphor 50 includes fluorescent substances, for example, YAG or BAM(BaMgAl₁₀O₁₇). These fluorescent substances absorb a part of the first, second, or third laser beams to emit light (fluorescence) from green to red. When the first, second, or third laser beams are applied to the phosphor 50, the green to red fluorescence from the phosphor 50 is combined with the first, second, or third laser beams, passed through the phosphor 50 without being absorbed, to produce pseudo white light.

Note that the phosphor 50 preferably has a substantially rectangular parallelepiped shape. The fluorescent substances may be formed into the substantially rectangular parallelepiped shape using binder. A mixture of resin, such as inorganic glass, and the fluorescent substances may be formed into the substantially rectangular parallelepiped shape. The phosphor 50 is also referred to as Micro White (or MW, registered trade mark).

An optical system such as an objective lens unit (not shown) is provided behind the capture window 42. The objective lens unit captures image light of the region of interest. Behind the objective lens unit, an image sensor 60 such as a CCD (Charge Coupled Device) is provided. The image sensor 60 receives the image light of the region of interest to image it. Note that an IT (interline transfer) type CCD is used as the image sensor 60. Alternatively, a CMOS (Complementary Metal-Oxide Semiconductor) with a global shutter may be used.

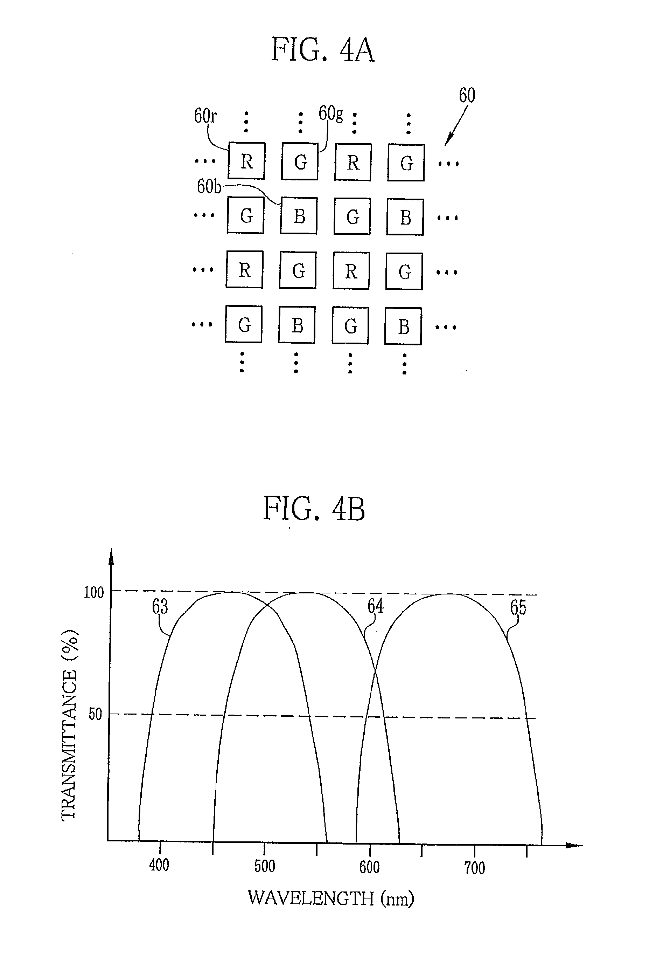

A light receiving surface (imaging surface) of the image sensor 60 receives the light from the objective lens unit. The image sensor 60 photoelectrically converts the received light into an image signal (analog signal) and outputs the analog image signal. Here, a color CCD is used as the image sensor 60. As shown in Fig. 4A, a pixel group in sets of B, G, and R pixels 60b, 60g, and 60r is arranged in a matrix array in the light receiving surface of the image sensor 60. The B pixel 60b is provided with a B color filter. The G pixel 60g is provided with a G color filter. The R pixel 60r is provided with an R color filter. The B, G, and R color filters exhibit spectral transmittance in blue, green, and red bands as shown by curves 63, 64, and 65, respectively, in Fig. 4B.

As shown in Fig. 2, the image sensor 60 generates a color image signal (analog image signal) composed of a red signal, a green signal, and a blue signal. The color image signal is inputted to an A/D converter 68 through a scope cable 67. The A/D converter 68 converts each color signal into a binary number, being a digital color image signal, in accordance with the voltage level of the color signal. The digital color image signal (hereinafter simply referred to as the image signal) is inputted to the processor device 13 through the connector 36.

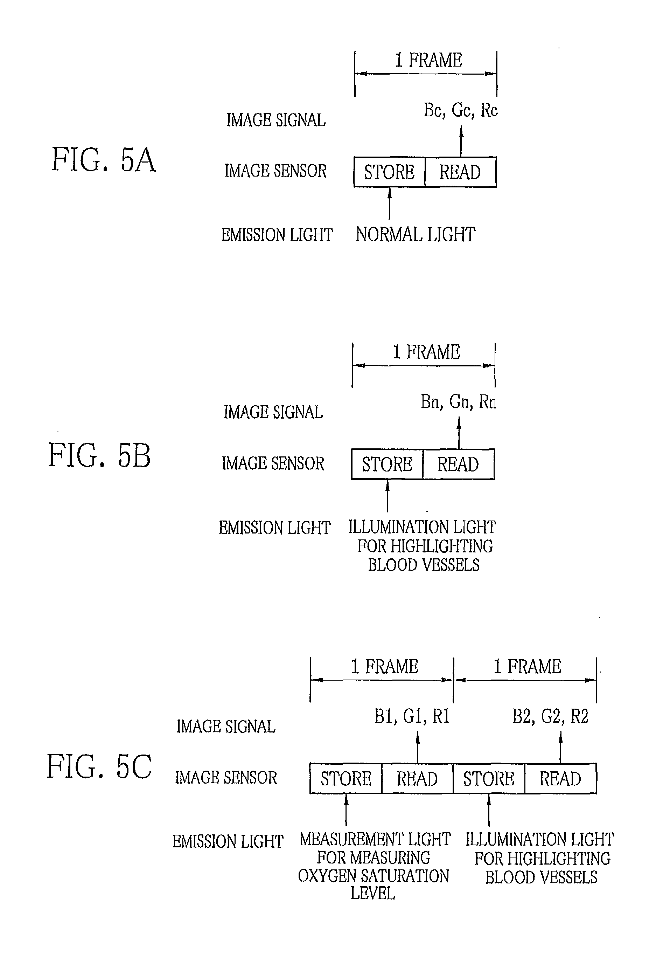

An imaging controller 70 controls imaging of the image sensor 60. The imaging controller 70 controls the image sensor 60 differently based on the mode selected. In the normal mode, as shown in Fig. 5A, a storing step and a reading step are performed in one frame period. In the storing step, the image sensor 60 photoelectrically converts the normal light into a charge and stores it. In the reading step, a blue signal Bc, a green signal Gc, and a red signal Rc are read from the B, G, and R pixels of the image sensor 60, respectively. The storing step and the reading step are repeated alternately in the normal mode. As shown in Fig. 5B, in the narrowband mode, a storing step and a reading step are performed in one frame period. In the storing step, the image sensor 60 photoelectrically converts the illumination light for highlighting blood vessels (highlighting light) into a charge and stores it. In the reading step, a blue signal Bn, a green signal Gn, and a red signal Rn are read from the B, G, and R pixels of the image sensor 60, respectively. The storing step and the reading step are repeated alternately in the narrowband mode.

As shown in Fig. 5C, in the narrowband image plus oxygen saturation mode, a storing step and a reading step are performed in each of a first frame and a second frame. In the storing step of the first frame, the image sensor 60 photoelectrically converts the measurement light for measuring oxygen saturation level into a charge and stores it. In the reading step of the first fame, a blue signal B1, a green signal G1, and a red signal R1 are read from the B, G, and R pixels of the image sensor 60, respectively. In the storing step of the second frame, the image sensor 60 photoelectrically converts the highlighting light into a charge and stores it. In the reading step of the second frame, a blue signal B2, a green signal G2, and a red signal R2 are read from the B, G, and R pixels of the image sensor 60, respectively. The imaging control of two frames is repeated in the narrowband image plus oxygen saturation mode.

The processor device 13 comprises a controller 71, an image processor 72, and a memory 74. The display device 14 and the input device 15 are connected to the controller 71. The controller 71 controls each section of the processor device 13. Based on information inputted from the changeover switch 21 of the endoscope device 12 or the input device 15, the controller 71 controls operation of each of the light source controller 20 of the light source device 11, the imaging controller 70 of the endoscope device 12, and the display device 14.

As shown in Fig. 2, the image processor 72 comprises a normal image processing section 80, a vessel image enhancement processing section 81, and an oxygen saturation plus vessel image enhancement processing section 82. The normal image processing section 80 assigns the blue signal Bc, the green signal Gc, and the red signal Rc, all acquired in the normal mode, to respective B, G, and R channels of the display device 14. Thereby, a normal image is displayed on the display device 14.

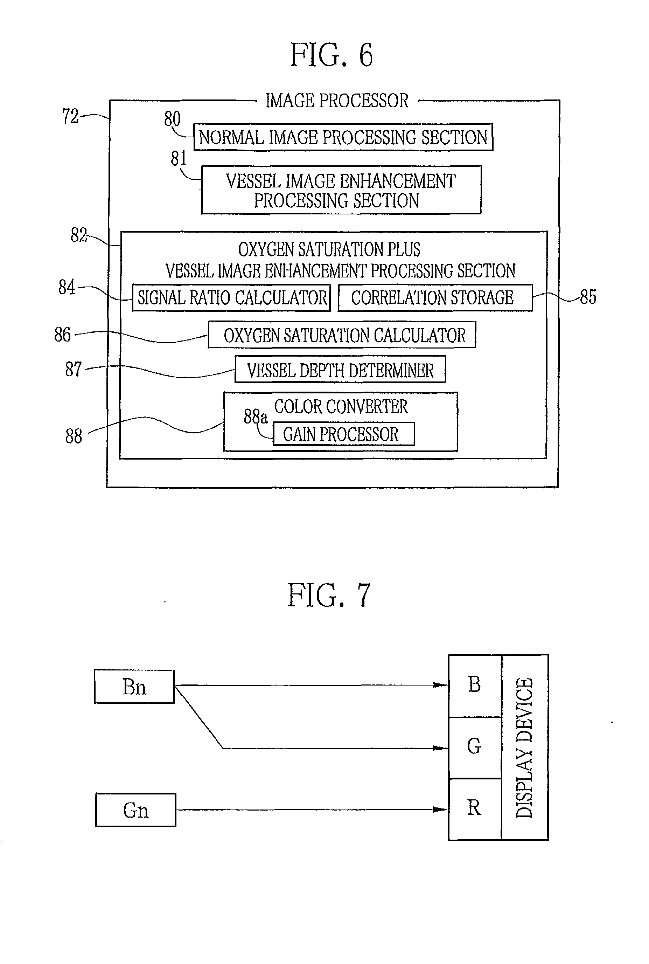

As shown in Fig. 7, out of the image signals acquired in the narrowband mode, the vessel image enhancement processing section 81 assigns the blue signal Bn to the B and G channels of the display device 14. The vessel image enhancement processing section 81 assigns the green signal Gn to the R channel of the display device 14. Thereby, the high-contrast vessel image is displayed in pseudo color on the display device 14. In the high-contrast vessel image, the surface blood vessels are colored "brown" and the subsurface blood vessels are colored "cyan".

As shown in Fig. 6, the oxygen saturation plus vessel image enhancement processing section 82 comprises a signal ratio calculator 84, correlation storage 85, an oxygen saturation calculator 86, a vessel depth determiner 87, and a color converter 88. The signal ratio calculator 84 calculates a signal ratio B1/G2 between the blue signal B1 and the green signal G2 and a signal ratio R2/G2 between the red signal R2 and the green signal G2, out of the image signals acquired in the narrowband image plus oxygen saturation mode. The signal ratio calculator 84 calculates a signal ratio between corresponding pixels of the color signals. The signal ratio calculator 84 calculates a signal ratio for every pixel in an image signal. Note that the signal ratio may be calculated only for the pixels in a vascular portion of the image signal. The vascular portion is identified based on a difference between an image signal of the vascular portion and an image signal of a portion other than the vascular portion.

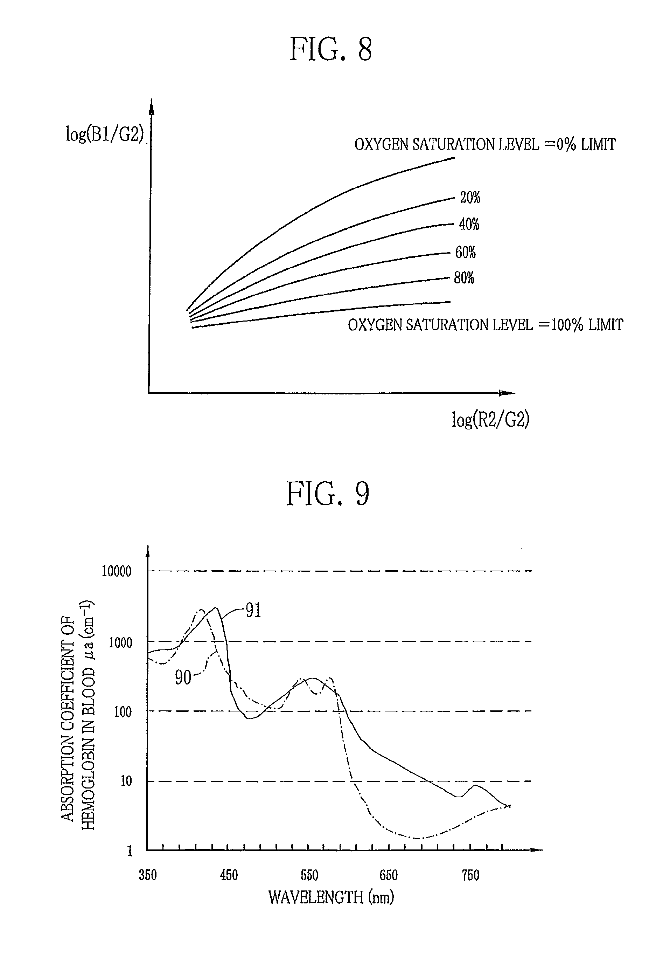

The correlation storage 85 stores a correlation between the signal ratios B1/G2 and R2/G2 and the oxygen saturation level. As shown in Fig. 8, the correlation is stored in a two-dimensional table in which contour lines of the oxygen saturation levels are defined in a two dimensional space. The positions and shapes of the contour lines are obtained from physical simulation of light scattering, and vary according to a blood volume. For example, a space between the contour lines increases or decreases with a change in the blood volume. Note that the signal ratios B1/G2 and R2/G2 are stored in log scale.

The above-described correlation is closely related to light absorption characteristics and light scattering characteristics of oxyhemoglobin and deoxyhemoglobin shown in Fig. 9. Here, a curve 90 shows absorption coefficient of oxyhemoglobin. A curve 91 shows absorption coefficient of deoxyhemoglobin. It is easy to obtain information on the oxygen saturation level using the light at 473 nm, for example, at which a difference between the absorption coefficient of the oxyhemoglobin and the absorption coefficient of the deoxyhemoglobin is large. However, the blue signal B1 including a signal component corresponding to the light at 473 nm is highly dependent on both the oxygen saturation level and the blood volume. To accurately calculate the oxygen saturation level not depending on or influenced by the blood volume, the signal ratios B1/G2 and R2/G2 are used. The signal ratios B1/G2 and R2/G2 are calculated using the blue signal B1, the red signal R2, and the green signal G2. The red signal R2 varies depending mainly on the blood volume. The green signal G2 is a reference signal (signal for standardization) for the blue signal B1 and the red signal R2.

The light in a wavelength range of 470 to 700 nm exhibits a small scattering coefficient in mucosal tissue and low wavelength dependence. With the use of the light in this wavelength range as the illumination light, blood information including information on a blood volume and information on an oxygen saturation level is obtained while influence caused by the depth of blood vessels is reduced.

Note that the correlation storage 85 may also store a correlation between the signal ratio R2/G2 and the blood volume. The correlation is stored in a one-dimensional table in which the blood volume increases as the signal ratio R2/G2 increases. The correlation between the signal ratio R2/G2 and the blood volume is used for calculating the blood volume.

The following is observed from wavelength-dependent characteristics of the absorption coefficient of hemoglobin in blood.
1. In a wavelength range close to 470 nm (for example, in a blue wavelength range with the center wavelength of 470 nm ± 10 nm), the absorption coefficient varies significantly in accordance with a change in the oxygen saturation level.
2. When averaged in a green wavelength range from 540 nm to 580 nm, the absorption coefficient is likely to be unaffected by the oxygen saturation level.
3. In a red wavelength range from 590 nm to 700 nm, the absorption coefficient appears to vary significantly in accordance with a change in the oxygen saturation level. Actually, however, the absorption coefficient is likely to be unaffected by the oxygen saturation level because the value of the absorption coefficient is extremely small.

As shown in Fig. 8, the signal ratio B1/G2 increases as the signal ratio R2/G2 increases (namely, the contour line of the oxygen saturation level = 0% limit extends toward the upper right direction). This is because the blood volume and the signal ratio R2/G2 correlate with each other as described above. The blood volume increases as the signal ratio R2/G2 increases. Of the signal values of the signals B1, G2, and R2, the signal value of the green signal G2 decreases the most, followed by the signal value of the blue signal B1, when the blood volume increases. This is because the absorption coefficient of the wavelength component (540 to 580 nm) in the green signal G2 is higher than that of the wavelength component (around 470 nm) in the blue signal B1 (see Fig. 9). Accordingly, in the signal ratio B1/G2, a decrease in the signal value G2 (denominator) is greater than that in the signal value B1 (numerator) as the blood volume increases. Namely, the signal ratio B1/G2 increases as the blood volume increases.

The oxygen saturation calculator 86 calculates an oxygen saturation level in each pixel based on the correlation stored in the correlation storage 85 and the signal ratios B1/G2 and R2/G2 calculated by the signal ratio calculator 84. Note that, hereinafter, luminance values of the corresponding pixels in the blue signal B1, the green signal G2, and the red signal R2, used for calculation of the oxygen saturation level, are referred to as B1^{*}, G2^{*}, and R2^{*}. The signal ratios calculated by the signal ratio calculator 84 are represented as B1^{*}/G2^{*} and R2^{*}/G2^{*}.

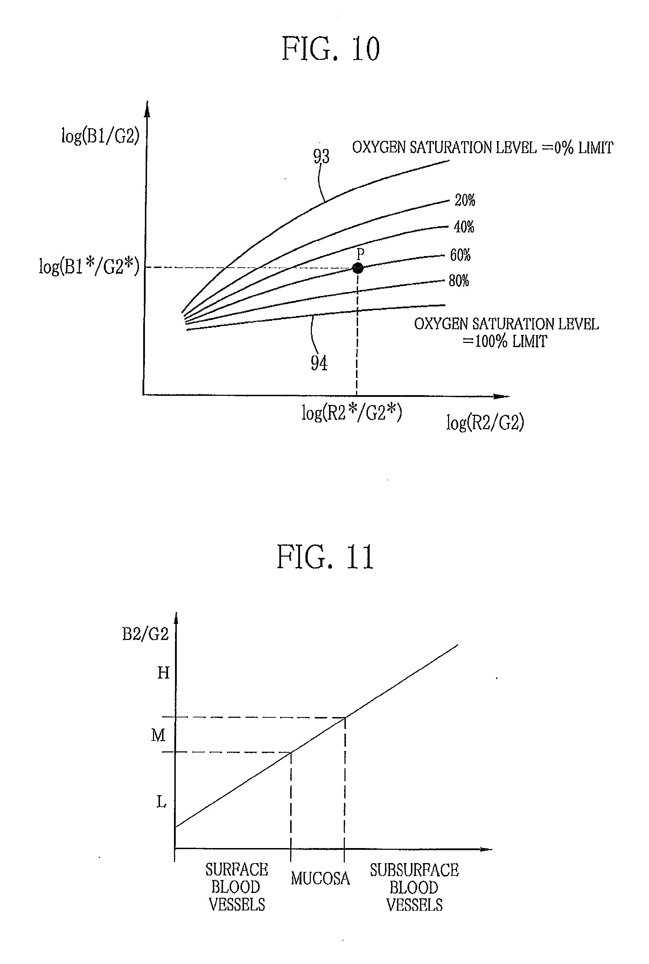

As shown in Fig. 10, the oxygen saturation calculator 86 determines a point P, corresponding to the signal ratios B1^{*}/G2^{*} and R2^{*}/G2^{*}, from the correlation stored in the correlation storage 85. When the point P is located between a lower limit line 93 (oxygen saturation level = 0% limit) and an upper limit line 94 (oxygen saturation level = 100% limit), the oxygen saturation level is the percentage expressed by the contour line on which the point P is located. For example, in Fig. 10, the point P is located on the contour line of "60%", so that the oxygen saturation level is 60%.

If the point P is located outside of the range between the lower limit line 93 and the upper limit line 94, for example, when the point P is located above the lower limit line 93, the oxygen saturation level is determined to be 0%. When the point P is located below the upper limit line 94, the oxygen saturation level is determined to be 100%. Note that when the point P is located outside of the range between the lower limit line 93 and the upper limit line 94, the reliability of the oxygen saturation level in the pixel may be reduced so as not to display the oxygen saturation level on the display device 14.

Based on the signal ratio B2/G2 between the blue signal B2 and the green signal G2, the vessel depth determiner 87 determines whether a pixel includes information of a surface blood vessel, information of mucosa, or information of a subsurface blood vessel. For the determination, a graph of Fig. 11 is used. The graph shows the correlation between the signal ratio B2/G2, information of surface blood vessel(s), information of mucosa, and information of subsurface blood vessel(s). The graph is stored as an LUT or the like in a memory (not shown) in advance. The vessel depth determiner 87 determines that the pixel includes the information of surface blood vessel (s) when the signal ratio B2/G2 is within a range L. The vessel depth determiner 87 determines that the pixel includes the information of mucosa when the signal ratio B2/G2 is within a range M higher than the range L. The vessel depth determiner 87 determines that the pixel includes the information of subsurface blood vessel(s) when the signal ratio B2/G2 is within a range H higher than the range M. Note that, instead of the signal ratio B2/G2, the signal ratio B1/G2 may be used to determine the depth of blood vessels.

The color converter 88 performs a color allocation process. In the color allocation process, the blue signal B2 is assigned or allocated to the B and G channels of the display device 14 and the green signal G2 is assigned or allocated to the R channel of the display device 14, out of the image signals acquired in the narrowband image plus oxygen saturation mode. When the vessel depth determiner 87 determines that the pixel corresponds to or includes the information of the surface or subsurface blood vessel, the color converter 88 performs the color allocation process on the pixel after a gain process is performed on the pixel. The gain process corresponds to the oxygen saturation level calculated by the oxygen saturation calculator 86. When the vessel depth determiner 87 determines that the pixel corresponds to the mucosa, the color converter 88 performs the color allocation process on the pixel without the gain process. With the gain process and the color allocation process, the high-contrast vessel image with oxygen saturation level is produced and displayed on the display device 14. The high-contrast vessel image with oxygen saturation level allows observation of information of the oxygen saturation level(s) while the surface and subsurface blood vessels are highlighted or enhanced.

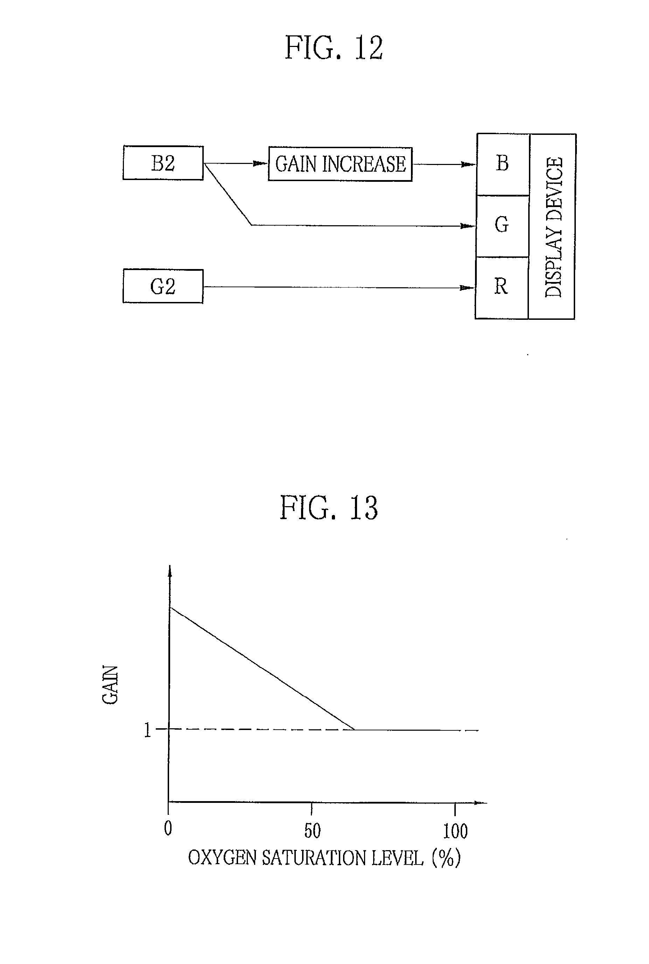

As shown in Fig. 12, a gain processor 88a performs the gain process on the pixel which includes the information of the surface blood vessel. To be more specific, in accordance with the oxygen saturation level, the gain processor 88a increases a gain of the blue signal B2 which is to be assigned or allocated to the B channel of the display device 14. The gain process is to multiply the signal value of the blue signal B2 by a predetermined gain. The correlation between the gain and the oxygen saturation level is stored in a memory (not shown) in advance. As shown in Fig. 13, when the oxygen saturation level is below a predetermined value (threshold value), the gain increases as the oxygen saturation level decreases (hypoxic or low oxygen condition). The gain process, however, is not performed on the blue signal B2 which is to be assigned to the G channel of the display device 14 and the green signal G2 which is to be assigned to the R channel of the display device 14. Note that, instead of the blue signal B2 which is to be assigned to the B channel, the gain of the blue signal B2 which is to be assigned to the G channel may be increased.

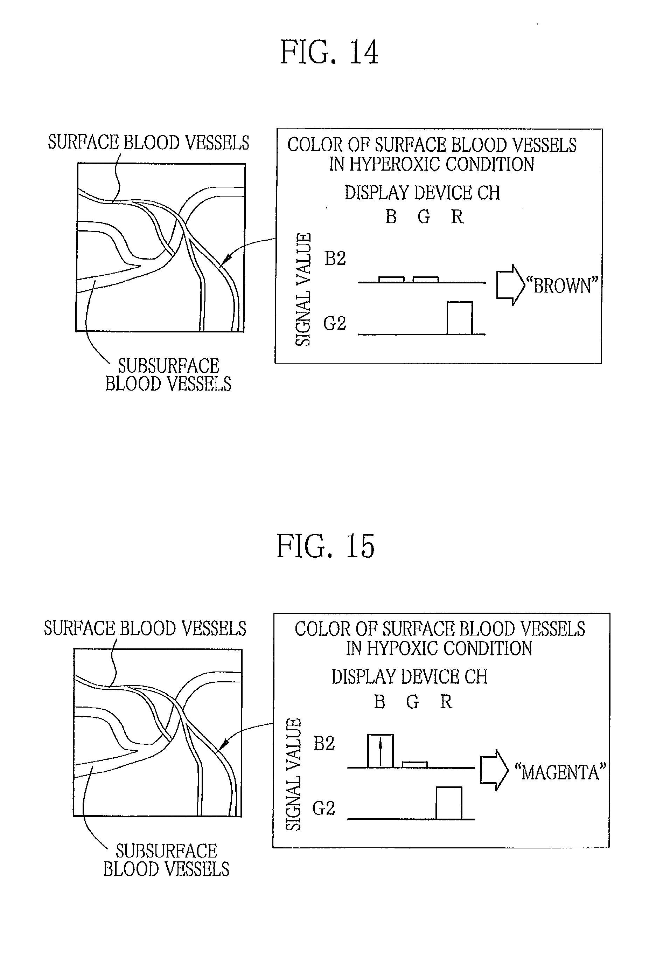

When the oxygen saturation level of the surface blood vessels is high, for example, in a range of 70 to 100%, the gain is "1", so that the signal value of the blue signal B2 which is to be assigned to the B channel of the display device 14 does not change. In this case, as shown in Fig. 14, the color of the displayed surface blood vessels is "brown", the same as that of the surface blood vessels in the high-contrast vessel image. As shown in Fig. 15, when the oxygen saturation level of the surface blood vessels is low, for example, less than 70%, the gain increases as the oxygen saturation level decreases. Hence, the signal value of the blue signal B2 to be assigned to the B channel of the display device 14 increases. The color of the displayed surface blood vessels changes to "magenta" as the oxygen saturation level decreases.

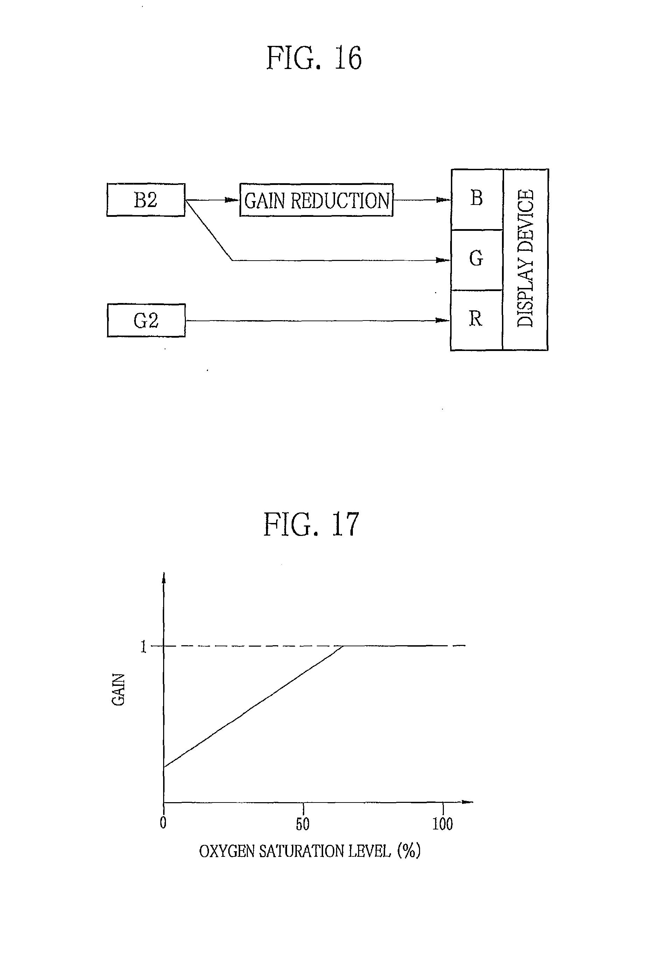

As shown in Fig. 16, the gain processor 88a performs the gain process on the pixel which corresponds to or includes the information of the subsurface blood vessel. To be more specific, the gain processor 88a reduces a gain of the blue signal B2 which is to be assigned or allocated to the B channel of the display device 14 in accordance with the oxygen saturation level. The gain process is performed by multiplying the blue signal B2 by a predetermined gain, in a manner similar to the gain process of the surface blood vessels. The correlation between the gain and the oxygen saturation level is stored in a memory (not shown) in advance. As shown in Fig. 17, when the oxygen saturation level is less than a predetermined value (threshold value), the gain decreases as the oxygen saturation level decreases (hypoxic condition). The gain process, however, is not performed on the blue signal B2 which is to be assigned to the G channel of the display device 14 and the green signal G2 which is to be assigned to the R channel of the display device 14. Note that, instead of the blue signal B2 to be assigned to the B channel, the gain of the blue signal B2 to be assigned to the G channel may be reduced.

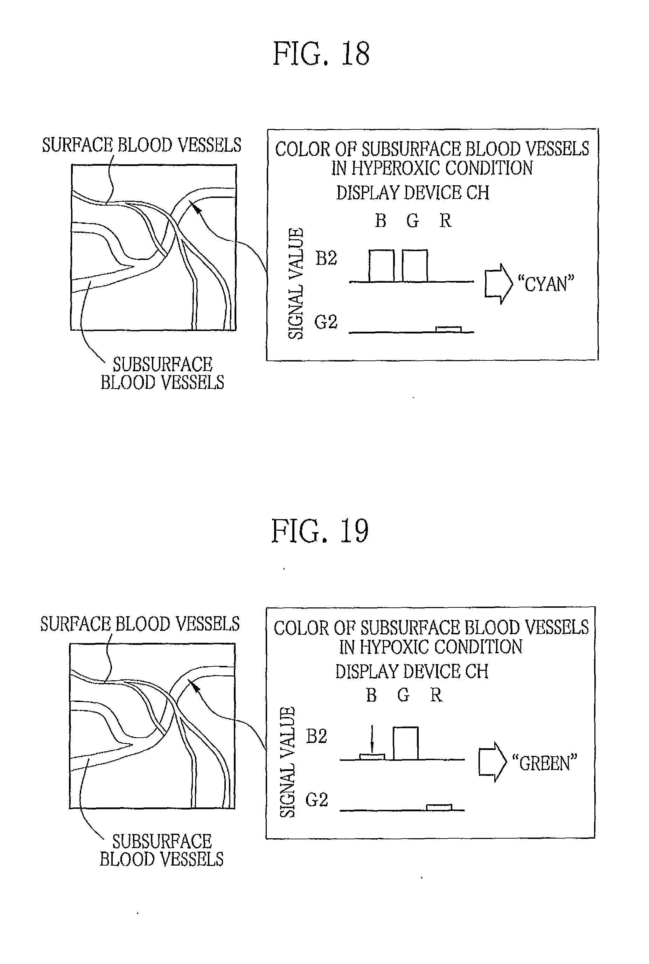

When the oxygen saturation level of the subsurface blood vessels is high, for example, in a range of 70 to 100%, the gain is "1", so that the signal value of the blue signal B2 which is to be assigned to the B channel of the display device 14 does not change. In this case, as shown in Fig. 18, the color of the displayed subsurface blood vessels is "cyan", the same as that of the subsurface blood vessels in the high-contrast vessel image. As shown in Fig. 19, when the oxygen saturation level of the subsurface blood vessels is low, for example, less than 70%, the gain decreases as the oxygen saturation level decreases. Hence, the signal value of the blue signal B2 to be assigned to the B channel of the display device 14 decreases. The color of the displayed subsurface blood vessels changes to "green" as the oxygen saturation level decreases.

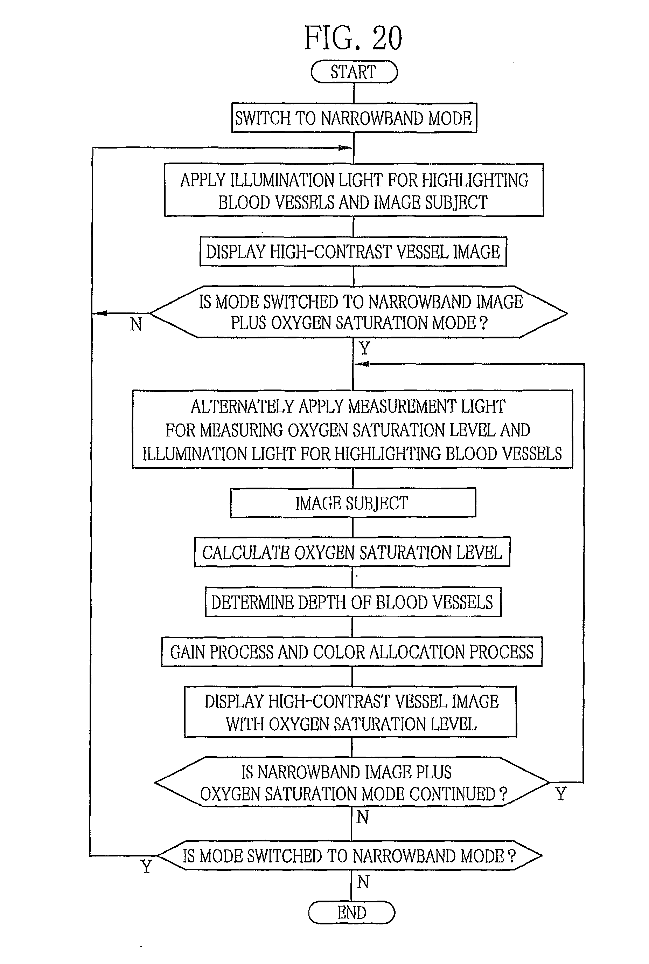

Next, steps in the narrowband mode and the narrowband image plus oxygen saturation mode are described with reference to a flowchart in Fig. 20. When the narrowband mode is chosen using the changeover switch 21 of the endoscope device 12, the highlighting light is applied to the subject. The color image sensor 60 captures the reflection image of the subject. Of the image signals acquired from the imaging, the blue signal Bn is assigned or allocated to the B and G channels of the display device 14. The green signal Gn is assigned to or allocated to the R channel of the display device 14. Thereby, the high-contrast vessel image is displayed in pseudo color. The displayed surface blood vessels are colored "brown". The displayed subsurface blood vessels are colored "cyan".

When a blood vessel pattern showing a sign of cancer appears on the high-contrast vessel image, the narrowband mode is switched to the narrowband image plus oxygen saturation mode. In the narrowband image plus oxygen saturation mode, the measurement light for measuring oxygen saturation level and the illumination light (highlighting light) for highlighting blood vessels is applied alternately to the subject. The color image sensor 60 images the subject under illumination of each light. The oxygen saturation level of hemoglobin in blood is calculated based on the blue signal B1, the green signal G2, and the red signal R2, out of the image signals acquired from the imaging.

Based on the signal ratio B2/G2, whether the pixel includes the information of a surface blood vessel, the information of mucosa, or the information of a subsurface blood vessel is determined. As for the pixel which includes the information of mucosa, the color allocation process is performed. In the color allocation process, the blue signal B2 is assigned or allocated to the B and G channels of the display device 14. The green signal G2 is assigned to the R channel of the display device 14. As for the pixel which includes the information of a surface blood vessel and the pixel which includes the information of a subsurface blood vessel, the gain process and the color allocation process are performed. The gain process corresponding to the oxygen saturation level is performed on the blue signal B2. Then, in the color allocation process, the blue signal B2 which has been subjected to the gain process is assigned to the B channel of the display device 14. The blue signal B2 which has not been subjected to the gain process is assigned to the G channel of the display device 14. The green signal G2 is assigned to the R channel of the display device 14. With the color allocation process and the gain process, the high-contrast vessel image with oxygen saturation level is produced and displayed on the display device 14. In the high-contrast vessel image with oxygen saturation level, colors of blood vessels vary in accordance with the oxygen saturation levels.

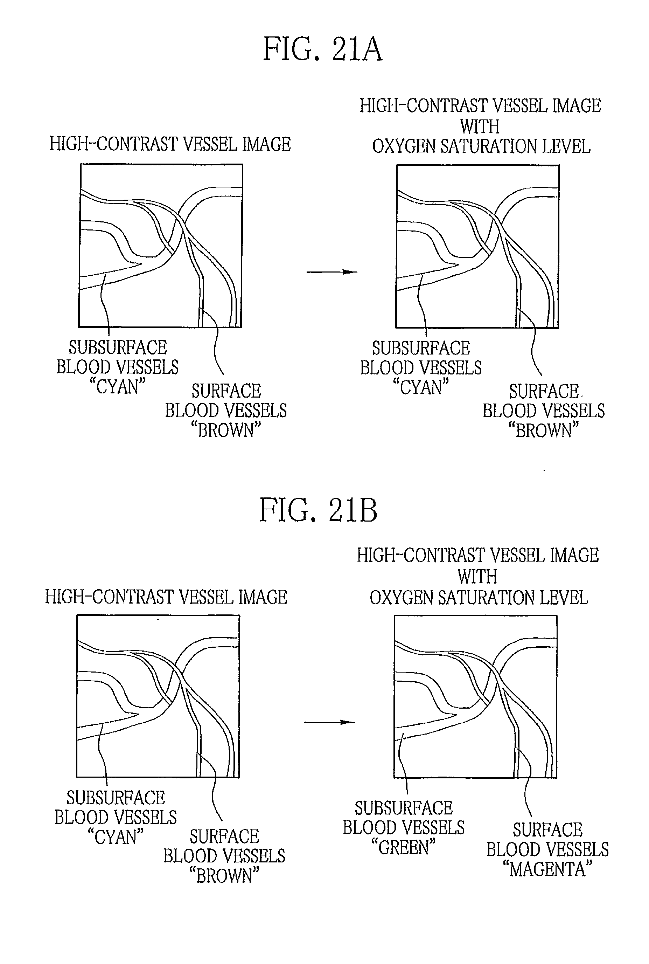

For example, as shown in Fig. 21A, when the narrowband mode is switched to the narrowband image plus oxygen saturation mode, the color of the displayed surface blood vessels remains "brown" and the color of the displayed subsurface blood vessels remains "cyan" if the oxygen saturation level is high. If the oxygen saturation level is low, as shown in Fig. 21B, the color of the displayed surface blood vessels changes from "brown" to "magenta" and the color of the displayed subsurface blood vessels changes from "cyan" to "green" when the narrowband mode is switched to the narrowband image plus oxygen saturation mode. When a blood vessel pattern visible in the narrowband mode is inadequate for determining cancer, the narrowband mode is switched to the narrowband image plus oxygen saturation mode. Thereby, the information of the oxygen saturation level is intuitively recognized from the changes in the colors. This significantly improves diagnosing performance for cancer.

The narrowband image plus oxygen saturation mode continues unless it is switched using the changeover switch 21. When switched, the narrowband image plus oxygen saturation mode returns to the narrowband mode (or the normal mode).

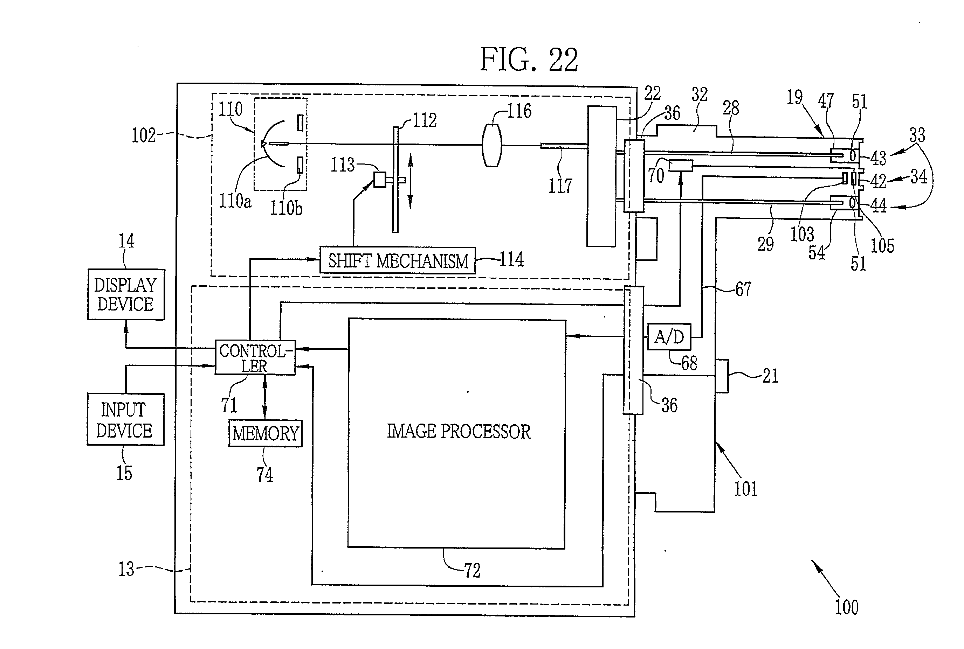

In the first embodiment, the illumination light from the semiconductor light source is used to illuminate the region of interest in the subject. In the second embodiment, a white light source such as a xenon lamp is used instead. A rotation filter or the like is used to separate light from broadband light from the white light source and the separated light is used to illuminate the region of interest (rotation filter method). In the second embodiment, an endoscope system 100 shown in Fig. 22 is used. A configuration of the endoscope system 100 is similar to that of the endoscope system 10, except for an endoscope device 101 and a light source device 102. Hereinafter, a configuration of each of the endoscope device 101 and the light source device 102 and parts related to them are described. Descriptions of parts other than those are omitted.

The endoscope device 101 differs from the endoscope device 12 in that the lighting section 33 of the distal portion is not provided with the phosphor 50. The light from the light source device 102 is applied to the region of interest through the light guides 28 and 29. Unlike the image sensor 60, an image sensor 103 is composed of a monochrome CCD with no color filter on the imaging surface. Other than that, the endoscope device 101 is similar to the endoscope device 12.

The light source device 102 is provided with a white light source 110, a rotation filter 112, a motor 113, and a shift mechanism 114. The white light source 110 emits broadband light BB (400 to 700 nm). The rotation filter 112 separates the broadband light BB into light of predetermined wavelengths. The motor 113 is connected to a rotation axis 112a of the rotation filter 112 to rotate the rotation filter 112 at a constant rotation speed. The shift mechanism 114 shifts the rotation filter 112 in its radial direction.

The white light source 110 is provided with a light source body 110a and an aperture stop 110b. The light source body 110a emits the broadband light BB. The aperture stop 110b changes or adjusts a light quantity of the broadband light BB. The light source body 110a is composed of a xenon lamp, a halogen lamp, or a metal halide lamp, for example. The size of the opening of the aperture stop 110b is controlled by a light quantity controller (not shown).

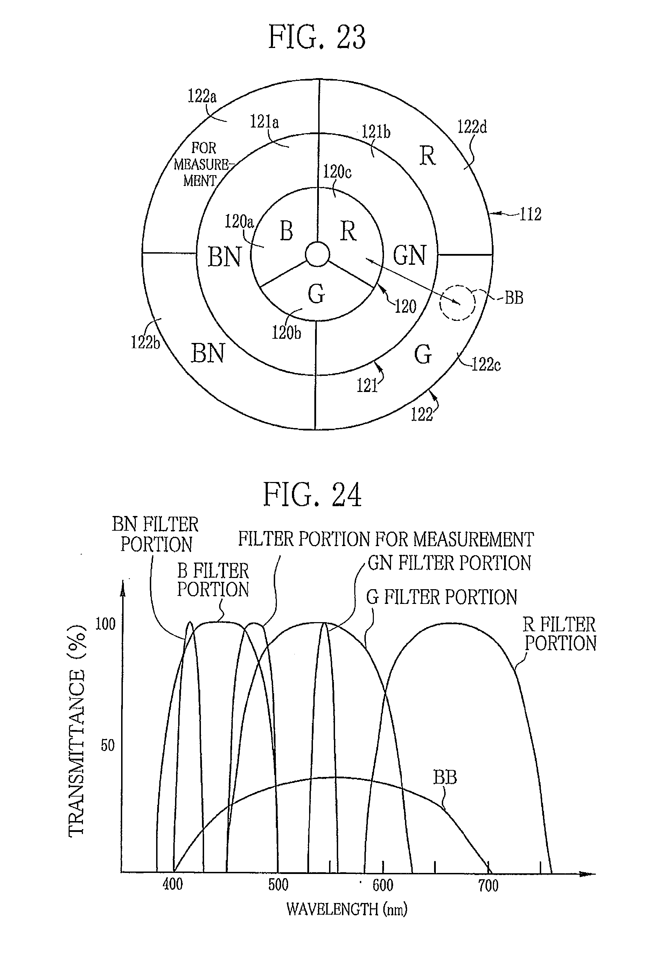

As shown in Fig. 23, the rotation filter 112 is rotated about the rotation axis 112a connected to the motor 113. The rotation filter 112 is provided with a first filter area 120, a second filter area 121, and a third filter area 122 disposed in this order in a radial direction from the center of rotation. In the normal mode, the first filter area 120 is set on an optical path of the broadband light BB. In the narrowband mode, the second filter area 121 is set on the optical path of the broadband light BB. In the narrowband image plus oxygen saturation mode, the third filter area 122 is set on the optical path of the broadband light BB. The shift mechanism 114 shifts the rotation filter 112 in its radial direction to place one of the first to third filter areas 120 to 122 on the optical path of the broadband light BB.

The first filter area 120 is provided with a B filter portion 120a, a G filter portion 120b, and an R filter portion 120c, each in a shape of a sector with a central angle of 120 degrees. As shown in Fig. 24, the B filter portion 120a transmits B light in the blue band (380 to 500 nm) out of the broadband light BB. The G filter portion 120b transmits G light in the green band (450 to 630 nm) out of the broadband light BB. The R filter portion 120c transmits R light in the red band (580 to 760 nm) out of the broadband light BB. The B light, the G light, and the R light passes through the rotation filter 112 sequentially in accordance with the rotation of the rotation filer 112. The B light, the G light, and the R light is incident sequentially on the light guides 28 and 29 through a condenser lens 116 and an optical fiber 117.

The second filter area 121 is provided with a BN filter portion 121a and a GN filter portion 121b, each in a shape of an annular sector with a central angle of 180 degrees. The BN filter portion 121a transmits blue narrowband light (Bn light) in a wavelength range of 400 to 420 nm with the center wavelength of 415 nm. The GN filter portion 121b transmits green narrowband light (Gn light) in a wavelength range of 530 to 550 nm with the center wavelength of 540 nm. The Bn light and the Gn light passes through the rotation filter 112 sequentially in accordance with the rotation of the rotation filer 112. The Bn light and the Gn light is incident sequentially on the light guides 28 and 29 through the condenser lens 116 and the optical fiber 117.

The third filter area 122 is provided with a measurement filter portion (denoted as "for measurement" in Fig. 23) 122a, a BN filter portion 122b, a G filter portion 122c, and an R filter portion 122d. The measurement filter portion 122a transmits the measurement light for measuring oxygen saturation level in a wavelength range of 450 to 500 nm out of the broadband light BB. Similar to the BN filter portion 121a, the BN filter portion 122b transmits the Bn light with the center wavelength of 415 nm. Similar to the G filter portion 120b, the G filter portion 122c transmits the G light in the green band (450 to 630 nm). Similar to the R filter portion 120c, the R filter portion 122d transmits the R light in the red band (580 to 760 nm). The measurement light for measuring oxygen saturation level, the Bn light, the G light, and the R light passes through the rotation filter 112 sequentially in accordance with the rotation of the rotation filer 112. The four types of light is incident sequentially on the light guides 28 and 29 through the condenser lens 116 and the optical fiber 117. Note that, it is preferable that the G filter portion 122c transmits the G light with the center wavelength of 540 nm.

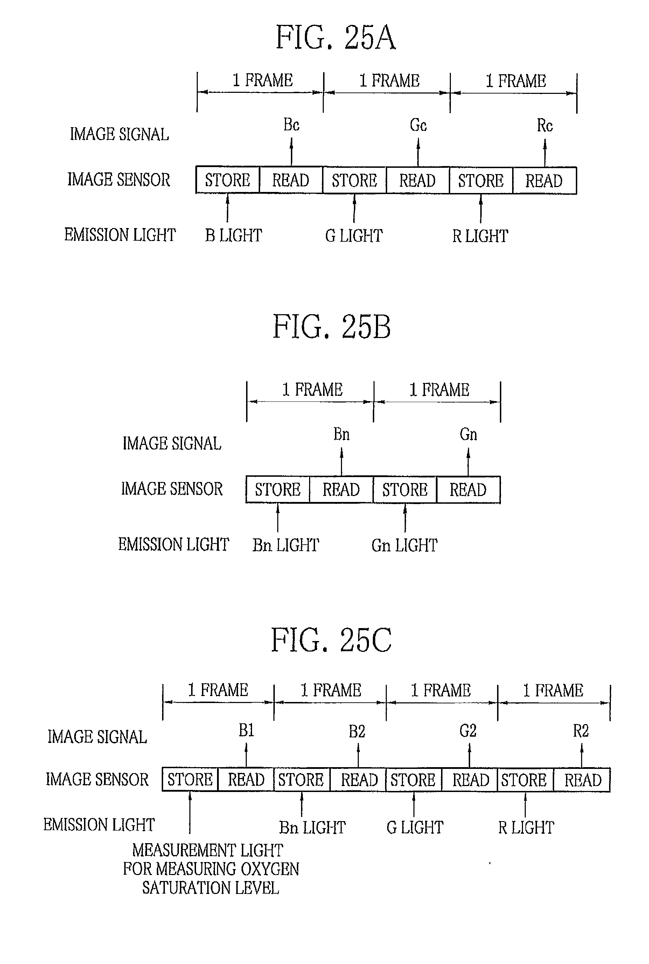

Because the endoscope system 100 of the second embodiment employs the rotation filter method, the imaging control in the endoscope system 100 differs from that in the endoscope system 10. In the normal mode, as shown in Fig. 25A, the image sensor 103 images the image light of each of three colors, the B, G, and R light, sequentially, and stores corresponding charges. Based on the respective stored charges, the image sensor 103 outputs a blue signal Bc, a green signal Gc, and a red signal Rc, sequentially. This procedure is repeated in the normal mode. The blue signal Bc, the green signal Gc, and the red signal Rc are assigned or allocated to the B channel, the G channel, and the R channel of the display device 14, respectively. Thereby the normal image is displayed on the display device 14.

In the narrowband mode, as shown in Fig. 25B, the image sensor 103 images the image light of each of two colors, the Bn light and the Gn light, sequentially and stores corresponding charges. Based on the respective stored charges, the image sensor 103 outputs a blue signal Bn and a green signal Gn sequentially. This procedure is repeated in the narrowband mode. The blue signal Bn is assigned to each of the B and G channels of the display device 14. The green signal Gn is assigned to the R channel of the display device 14. Thereby, the high-contrast vessel image is displayed on the display device 14.

In the narrowband image plus oxygen saturation mode, as shown in Fig. 25C, the image sensor 103 images each of the measurement light for measuring oxygen saturation level, the BN light, the G light, and the R light, sequentially and stores corresponding charges. Based on the respective stored charges, the image sensor 103 sequentially outputs a blue signal B1, a blue signal B2, a green signal G2, and a red signal R2. The procedure is repeated in the narrowband image plus oxygen saturation mode.

In a manner similar to the first embodiment, the oxygen saturation level of hemoglobin in blood is calculated based on the blue signal B1, the green signal G2, and the red signal R2, out of the image signals acquired in the narrowband image plus oxygen saturation mode. In a manner similar to the first embodiment, the depth of the blood vessel is determined based on the signal ratio B2/G2. The determination of the depth of blood vessel is to determine whether a pixel includes the information of a surface blood vessel, the information of mucosa, or the information of a subsurface blood vessel. Based on the result of the calculation of the oxygen saturation level and the result of the determination of the depth of blood vessel, the color allocation process and the gain process are performed in a manner similar to the first embodiment. Thereby, the high-contrast vessel image with oxygen saturation level is displayed on the display device 14. Note that it is preferable to improve contrast of the subsurface blood vessels (for example, through a frequency filtering process for medium frequency components) because the green signal G2 is generated based on the broadband G light.

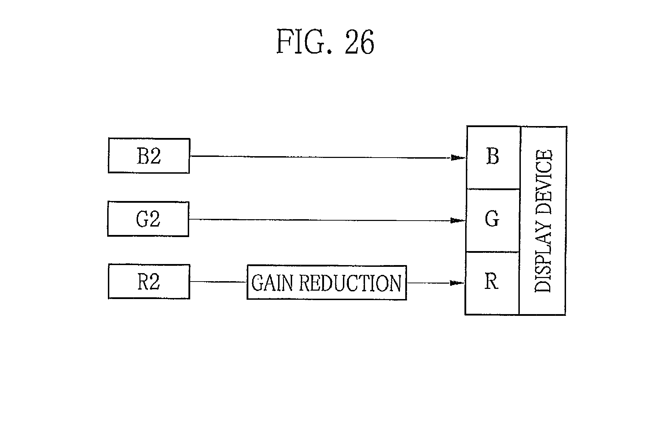

In the first and second embodiments, in the narrowband image plus oxygen saturation mode, the blue signal B2 is assigned to each of the B and G channels of the display device 14. The green signal G2 is assigned to the R channel of the display device 14. In a third embodiment, as shown in Fig. 26, the blue signal B2, the green signal G2, and the red signal R2 are assigned to the B, G, and R channels of the display device 14, respectively. Thereby the subject is displayed in colors similar to those of the subject illuminated with the white light. In other words, unevenness of the surface of the body cavity is observable. In the third embodiment, the depths of blood vessels (whether the blood vessels are the surface blood vessels, the subsurface blood vessels, or in the mucosa) are not determined. In a gain process of the third embodiment, a gain of the red signal R2 of every pixel is reduced as the oxygen saturation level decreases. Note that the method for reducing the gain is similar to that in the first embodiment.

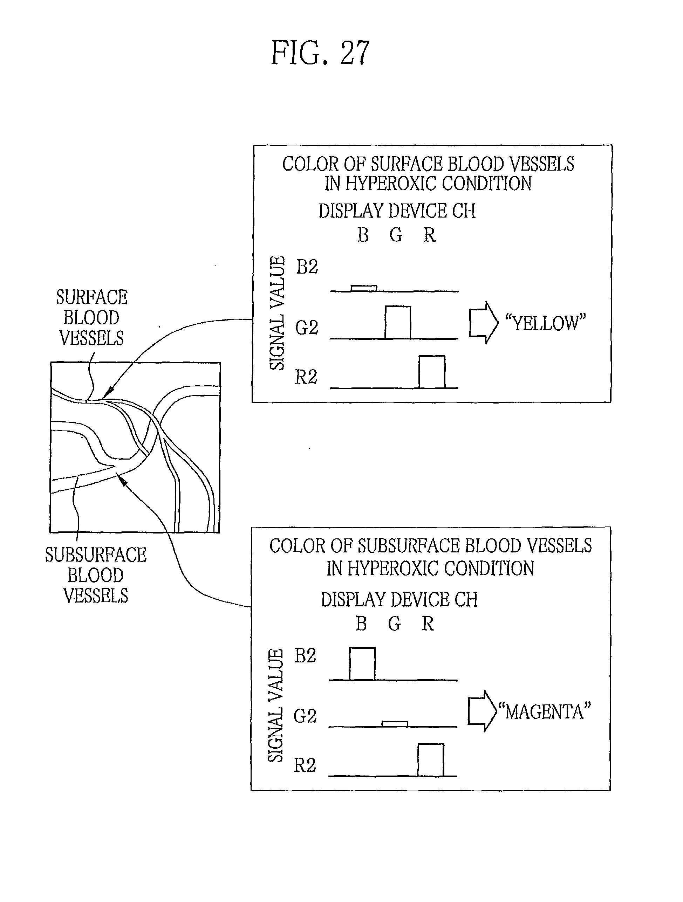

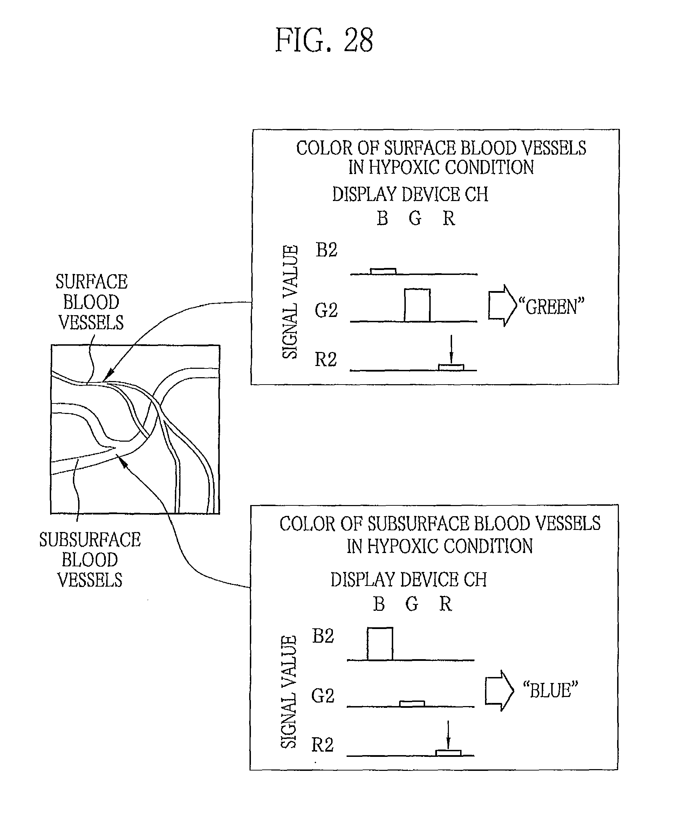

For example, as shown in Fig. 27, as for the surface blood vessels with a high oxygen saturation level, only the blue signal B2 has a low signal value. The green signal G2 and the red signal R2 have high signal values. Hence, the color of the displayed surface blood vessels is "yellow". As for the subsurface blood vessels with a high oxygen saturation level, only the green signal G2 has a low signal value. The blue signal B2 and the red signal R2 have high signal values. Hence the color of the displayed subsurface blood vessels is "magenta". As shown in Fig. 28, when the gain of the signal value of the red signal R2 is reduced as the oxygen saturation level decreases, the color of the displayed surface blood vessels with a low oxygen saturation level changes from "yellow" to "green". The color of the displayed subsurface blood vessels with a low oxygen saturation level changes from "magenta" to "blue".

Note that, in the first embodiment, the phosphor 50 is provided in the distal portion 19. Alternatively, the phosphor 50 may be provided in the light source device 11. In this case, the phosphor 50 is placed between the LD2 (445 nm) and the optical fiber 25. The phosphor 50 may not be placed between the LD1 (473 nm) and the optical fiber 24 and between the LD3 (405 nm) and the optical fiber 26.

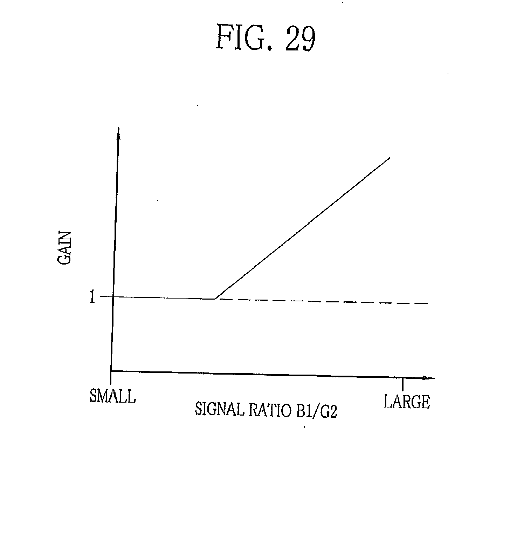

In the first and second embodiments, the oxygen saturation level is numerically calculated using the correlation stored in the correlation storage. The gain process is performed in accordance with the calculated oxygen saturation level. The gain process may be performed without calculating the oxygen saturation level. For example, the gain process is performed using a graph of Fig. 29, illustrating the relationship between the gain and the signal ratio B1/G2, to increase the gain of the blue signal B2 as shown in Fig. 12. The signal ratio B1/B2 changes with the oxygen saturation level. Generally, the signal ratio B1/G2 increases as the oxygen saturation level decreases. Hence, in the graph of Fig. 29, the gain increases as the signal ratio B1/G2 increases when the signal ratio B1/G2 exceeds a predetermined value (threshold value). Note that, in the case of reducing the gain in the gain process, the gain is reduced as the signal ratio B1/G2 increases when the signal ratio B1/G2 exceeds a predetermined value.

Note that, in the first and second embodiments, the gain process is performed on the pixels corresponding to the surface and subsurface blood vessels after the depths of the blood vessels are determined. Alternatively, the gain of every pixel may be increased or reduced without the determination of the depth of the blood vessels. When the gain is increased, only the colors of the surface blood vessels change as the oxygen saturation level decreases. When the gain is reduced, only the colors of the subsurface blood vessels change as the oxygen saturation level decreases.

Note that, in the first to third embodiments, the colors of the displayed blood vessels are changed by increasing or decreasing the gain (gain process). Alternatively, a color conversion program such as 2D-LUT or 3D-LUT may be used to change the color of the displayed blood vessels. In the first and second embodiments, the gain process is performed on the blue signal. In the third embodiment, the gain process is performed on the red signal. The gain process is not limited to the above. Any type of gain process may be used as long as the color of the displayed blood vessels at a specific depth is changed in accordance with a change in the oxygen saturation level. For example, the gain process may be performed on two of the blue signal, the green signal, and the red signal and not on the remaining image signal.

Note that, each of the endoscope systems of the first to third embodiments is provided with the normal mode, the narrowband image plus oxygen saturation mode, and the narrowband mode. In the narrowband mode, the high-contrast vessel image, being one type of narrowband images, is displayed. In addition, a normal image plus oxygen saturation mode may be provided. In the normal image plus oxygen saturation mode, an image in which color characteristic values of a normal image vary in accordance with the oxygen saturation levels is displayed. Note that the normal image plus oxygen saturation mode is switched to another mode using the changeover switch 21.

Note that, in the first to third embodiments, the oxygen saturation level is used to produce the high-contrast vessel image with the oxygen saturation level. The oxygen saturation level is a percentage of oxyhemoglobin relative to a blood volume that is the sum of the oxyhemoglobin and deoxyhemoglobin. Alternatively or in addition, oxyhemoglobin index or deoxyhemoglobin index may be used. The oxyhemoglobin index is calculated using an expression "blood volume × oxygen saturation level (%)". The deoxyhemoglobin index is calculated using an expression "blood volume × (100-oxygen saturation level) (%)".

Various changes and modifications are possible in the present invention and may be understood to be within the present invention.

## Claims

1. An endoscope system (10, 100) comprising:
a lighting means (33) for applying each of first illumination light and second illumination light on a frame-by-frame basis to a subject including a blood vessel, the first illumination light including at least one type of narrowband light, the second illumination light including a wavelength range in which an absorption coefficient varies with a change in oxygen saturation level of hemoglobin in blood;
an image signal acquisition means (60, 103) for imaging the subject, illuminated with the first illumination light, on the frame-by-frame basis to acquire a first color image signal and imaging the subject, illuminated with the second illumination light, on the frame-by-frame basis to acquire a second color image signal; and
a display control means (88, 88a) for producing a display image in which at least a first color signal, out of the first color image signal, is changed in accordance with the oxygen saturation level, the oxygen saturation level being obtained using at least the second color image signal; and
a display means (14) for displaying the display image.

2. The endoscope system of claim 1, wherein the at least one type of narrowband light is at least one of blue narrowband light and green narrowband light.

3. The endoscope system of claim 2, wherein
the first color image signal includes a blue signal, a red signal, and a green signal and one of the blue signal, the red signal, and the green signal is the first color signal; and
the display means has B, G, and R channels.

4. The endoscope system of claim 3, wherein the display control means changes the blue signal which is to be assigned to the B channel in accordance with the oxygen saturation level, and the blue signal which is to be assigned to the B channel is the first color signal, and the display control means does not change the blue signal which is to be assigned to the G channel and the green signal which is to be assigned to the R channel.

5. The endoscope system of claim 3, wherein the display control means changes the blue signal which is to be assigned to the G channel in accordance with the oxygen saturation level, and the blue signal which is to be assigned to the G channel is the first color signal, and the display control means does not change the blue signal which is to be assigned to the B channel and the green signal which is to be assigned to the R channel.

6. The endoscope system of claim 3, wherein the display control means changes the red signal which is to be assigned to the R channel in accordance with the oxygen saturation level, and the red signal which is to be assigned to the R channel is the first color signal, and the display control means does not change the blue signal which is to be assigned to the B channel and the green signal which is to be assigned to the G channel.

7. The endoscope system of claim 2 or 3, wherein the at least first color signal is two of the blue signal, the red signal, and the green signal.

8. The endoscope system of one of claims 1 to 7, further comprising an oxygen saturation calculation means (86) for calculating the oxygen saturation level from the second color image signal or the first and the second color image signals.

9. The endoscope system of one of claims 1 to 8, further comprising a vessel depth determination means (87) for determining a depth of a blood vessel in the subject based on the first or the second color image signal, the display control means changing the first color signal in accordance with the depth of the blood vessel and the oxygen saturation level to control the display image.

10. The endoscope system of one of claims 1 to 9, wherein the display control means changes the first color signal in accordance with the oxygen saturation level when the oxygen saturation level is less than a predetermined value or a standardization signal is out of a predetermined range, and the standardization signal is obtained by standardizing the second color image signal with the first color image signal.

11. The endoscope system of one of claims 1 to 10, further comprising a mode selection means (21) for selecting one of two modes, the two modes including at least one of a narrowband image plus oxygen saturation mode, a narrowband mode, and a normal image plus oxygen saturation mode, the display image being displayed in the narrowband image plus oxygen saturation mode, a narrowband image produced based on the first color image signal being displayed in the narrowband mode, an image in which a color characteristic value of a normal image is changed in accordance with the oxygen saturation level being displayed in the normal image plus oxygen saturation mode, the normal image being produced by imaging the subject illuminated with broadband third illumination light.

12. The endoscope system of one of claims 1 to 11, wherein
the image signal acquisition means has a color image sensor (60);
the first illumination light includes blue narrowband light of 440 to 460 nm, fluorescence obtained by converting the blue narrowband light of 440 to 460 nm with a wavelength converter (50), and blue narrowband light with a center wavelength of 400 to 410 nm; and
the second illumination light includes blue narrowband light of 460 to 480 nm and fluorescence obtained by converting the blue narrowband light of 460 to 480 nm with a wavelength converter (50).

13. The endoscope system of one of claims 1 to 11, wherein
the image signal acquisition means has a monochrome image sensor (103); and
the first illumination light includes blue light of 400 to 420 nm and green light of 530 to 550 nm applied alternately; and
the second illumination light is blue light of 450 to 500 nm.

14. A processor device (13) used in an endoscope system (10, 100), the endoscope system including a lighting device (33) and an endoscope device (12, 101), the lighting device applying each of first illumination light and second illumination light on a frame-by-frame basis to a subject including a blood vessel, the first illumination light including narrowband light, the second illumination light including a wavelength range in which an absorption coefficient varies with a change in oxygen saturation level of hemoglobin in blood, the endoscope device imaging the subject, illuminated with the first illumination light, on the frame-by-frame basis to acquire a first color image signal, the endoscope device imaging the subject, illuminated with the second illumination light, on the frame-by-frame basis to acquire a second color image signal, the processor device comprising:
a receiving means (72) for receiving the first and the second color image signals from the endoscope device; and
a display control means (88, 88a) for producing a display image in which at least a first color signal, out of the first color image signal, is changed in accordance with the oxygen saturation level obtained using at least the second color image signal and displaying the display image on a display means (14).

15. A method for controlling a display of an endoscope image, comprising the steps of:
applying each of first illumination light and second illumination light on a frame-by-frame basis to a subject including a blood vessel, the first illumination light including narrowband light, the second illumination light including a wavelength range in which an absorption coefficient varies with a change in oxygen saturation level of hemoglobin in blood,
imaging the subject, illuminated with the first illumination light, with an endoscope device (12, 101) on the frame-by-frame basis to acquire a first color image signal, and
imaging the subject, illuminated with the second illumination light, with the endoscope device on the frame-by-frame basis to acquire a second color image signal;
producing a display image in which at least a first color signal, out of the first color image signal, is changed in accordance with the oxygen saturation level, the oxygen saturation level being obtained using at least the second color image signal; and
displaying the display image on a display means (14).
